Europäisches Patentamt

European Patent Office

Office européen des brevets

(11) Publication number: **0 368 288**
**A2**

## (12) EUROPEAN PATENT APPLICATION

(21) Application number: **89120719.3**

(22) Date of filing: **08.11.89**

(51) Int. Cl.5: **C07B 33/00, B01J 19/00**

(30) Priority: **09.11.88 US 268959**

(43) Date of publication of application:
**16.05.90 Bulletin 90/20**

(84) Designated Contracting States:
**BE DE ES FR GB IT**

(71) Applicant: **UNION CARBIDE CORPORATION**
**39 Old Ridgebury Road**
**Danbury Connecticut 06817(US)**

(72) Inventor: **Daubler, Leonard Michael**
**2701 Indian Forest Trail**
**Helena Alabama 35080(US)**

(74) Representative: **Schwan, Gerhard, Dipl.-Ing.**
**Elfenstrasse 32**
**D-8000 München 83(DE)**

(54) **Method for carrying out an oxidation reaction.**

(57) A method for supplementing the oxygen flow to an industrial scale oxidation reaction employing compressed air as the major source of oxygen for the reaction wherein liquid oxygen is supplied into and vaporized within the feed air stream to the compressor upstream of the compressor to supply additional oxygen to the air and to cool and contract the air upstream of the compressor.

EP 0 368 288 A2

# METHOD FOR CARRYING OUT AN OXIDATION REACTION

## Technical Field

This invention relates generally to industrial scale oxidation reactions and more particularly to such oxidation reactions wherein air is compressed in a compressor and then passed in contact with an oxidizable specie to provide oxygen for the reaction.

## Background Art

Many industrial scale oxidation reactions require large amounts of oxygen to be effectively carried out. When nitrogen and other components of air are not detrimental to the oxidation reaction, an effective source of oxygen for such oxidation reactions is the ambient air. Generally such air is compressed in a compressor and then passed under pressure into a reaction vessel. The air bubbles through liquid in the vessel or mixes with gas in the vessel and in the process oxygen in the air comes in contact with and reacts with oxidizable components in the vessel.

A problem which arises in such oxidation reaction systems is the reduction of the oxygen throughput during hot weather such as during the summer months of the northern latitudes. As is known, the ambient air expands with a temperature rise or with a pressure drop resulting in fewer molecules, including oxygen molecules, in a given volume. During some weather conditions this reduction in oxygen molecule concentration disrupts or otherwise detrimentally effects the oxidation reaction.

Those skilled in this art have addressed this problem by providing supplemental oxygen gas to the reaction vessel during such weather periods. Typically the supplemental oxygen is stored on site in liquid form, passed through a vaporizer to be vaporized to gas, and then passed into the reaction vessel. While such supplemental oxygen gas systems are effective, they entail significant capital and operating costs due to the extra equipment required and the added piping and flow control necessary to ensure efficient oxygen usage. This is especially the case when the oxidation reaction exceeds about 275 pounds per square inch absolute (psia). Above this pressure specialized equipment and flow control systems are generally necessary.

Accordingly it is an object of this invention to provide a method for carrying out an oxidation reaction wherein supplemental oxygen may be supplied to the oxidation reaction without need for a separate oxygen supply system.

## Summary of the Invention

The above and other objects which will become apparent to those skilled in the art upon a reading of this disclosure are attained by the present invention which is:

A method for carrying out an oxidation reaction comprising:

(a) providing an air stream to a compressor;

(b) providing liquid oxygen into the air stream upstream of the compressor;

(c) vaporizing the liquid oxygen upstream of the compressor by heat exchange with the air to provide additional oxygen to the air and to cool and contract the air;

(d) compressing the cooled, contracted, oxygen enriched air by passage through the compressor;

(e) passing the compressed, oxygen-enriched air in contact with a fluid having at least one oxidizable component; and

(f) reacting vaporized oxygen and oxygen from the air with oxidizable component(s) to carry out an oxidation reaction.

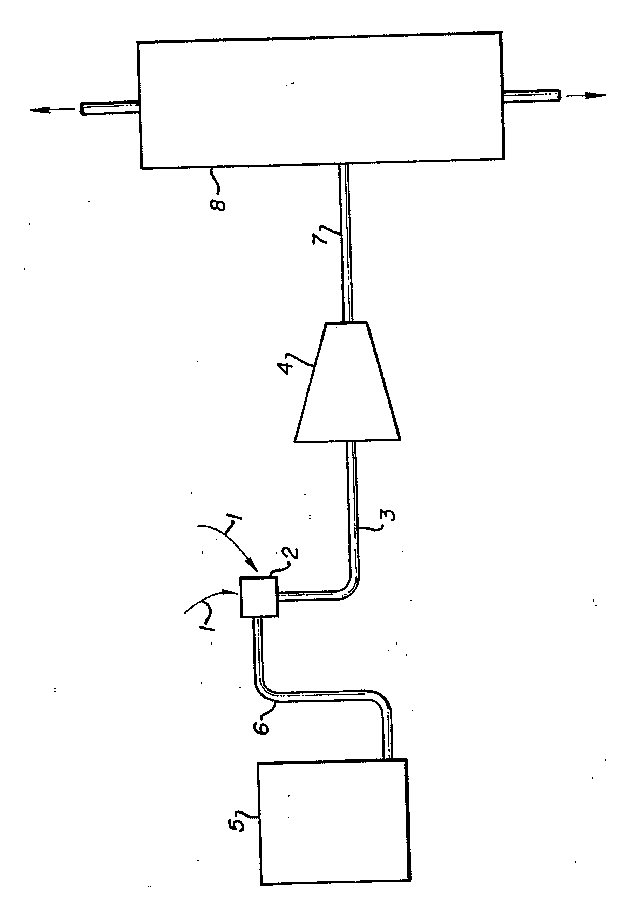

## Brief Description of the Drawing

The sole Figure is a schematic representation of one preferred arrangement useful for carrying out the method of this invention.

## Detailed Description

The invention will be described in detail with reference to the Drawing.

Referring now to the Figure, ambient air 1 is drawn into intake 2 through filter elements in the walls of intake 2. The air stream is passed from intake 2 through line 3 and into compressor 4. Any effective type of compressor may be employed in the practice of this invention. Among such types one can name axial, centrifugal and reciprocating compressors.

Liquid oxygen is supplied into the air stream upstream of the compressor. The liquid oxygen may be stored on-site in tank 5 and passed through conduit 6 into the air stream. The liquid oxygen may be supplied to the air stream at any point upstream of the compressor. In the Figure the liquid oxygen is shown as being supplied to the air stream within intake 2. The liquid oxygen may also be supplied to the air stream within conduit 3. It is desirable that the liquid oxygen be supplied to the air stream so that the oxygen and air form a substantially uniformly mixed stream when the stream reaches the compressor.

The liquid oxygen is vaporized upstream of the compressor by heat exchange with the air. The heat exchange may be direct or indirect. As used herein the term "direct heat exchange" means the bringing of two fluids into heat exchange relation with physical contact or intermixing of the fluids with each other, and the term "indirect heat exchange" means the bringing of two fluids into heat exchange relation without physical contact or intermixing of the fluids with each other.

It is important to the practice of this invention that the liquid oxygen vaporize substantially completely before passage into the compressor. Thus the flowrate of the liquid oxygen into the air stream should be such that substantially all of the liquid oxygen is vaporized upstream of the compressor. Furthermore it is undesirable to pass water condensed out of the air into the compressor. Thus the amount of liquid oxygen passed into the air stream should be such that the air stream temperature is not reduced below the dew point or, if water is condensed out of the air stream, the water should be removed from the air stream prior to its passage into the compressor.

The vaporization of the liquid oxygen within the air stream results in the simultaneous occurrence of two beneficial and complementary effects. First, the vaporized oxygen provides additional oxygen molecules to the air flowing to the compressor and thus serves as a source of additional oxygen molecules for the downstream oxidation reaction which is advantageous during weather conditions when the number of oxygen molecules per given volume of air is reduced. Second, the vaporization of the liquid oxygen serves to cool the incoming air, generally by about 8 to 12 °F per one percent oxygen enrichment. This causes the incoming air to contract thus increasing the number of air oxygen molecules per unit volume. Thus even more oxygen molecules can be supplied to the oxidation reaction for any given amount of compression.

The cooled, compressed, oxygen-enriched air, which contains an increased concentration of oxygen molecules from both the vaporized liquid oxygen and the contracted feed air, is then passed from compressor 4 through conduit 7 into reaction vessel 8 where oxidation is carried out.

Reaction vessel 8 contains fluid which may be liquid, gas or a mixture thereof. The fluid contains at least one oxidizable component. Often the reaction vessel will also contain a catalyst to promote the oxidation. The compressed fortified air passes in contact with the contents of the reaction vessel and oxygen reacts with the oxidizable component-(s) to carry out the oxidation.

Among the many industrial scale oxidation reactions which can be carried out with the method of this invention one can name the oxidation of para-xylene to produce terephthalic acid, the partial oxidation of hydrocarbons to produce synthesis gas, the oxidation of light aliphatic hydrocarbons to produce acetic acid, methanol, formaldehyde or acetaldehyde, the oxidation of ethylene to produce ethylene oxide, acetaldehyde or vinyl acetate, the oxidation of propylene to produce propylene oxide, the oxidation of paraffin wax to produce fatty acids, the oxidation of wastewater, the oxidation of fuel in a combustion reaction, and oxidation in a bioreaction such as respiration. The products of the reaction or reactions within reaction vessel 8 may be separated and recovered using conventional separation and recovery techniques well known to those skilled in the art.

As can be seen the method of this invention differs from and is in improvement over conventional supplemental oxygen systems. The method of this invention increases the feed rate of oxygen molecules to the oxidation reaction by adding oxygen to the reaction. However the method of this invention simultaneously increases the feed rate of oxygen molecules to the oxidation reaction by causing the oxygen molecules in the incoming feed air to increase per given volume of air. The net result is that less supplemental oxygen need be supplied to the oxidation reaction for any given production rate. Moreover, the method of this invention requires less capital cost than a conventional supplemental oxygen system and also reduces operating costs. Moreover, where the oxidation reaction is carried out above the critical pressure of liquid oxygen, the method of this invention is particularly advantageous in that it allows for the elimination of much of the expensive specialized oxygen delivery apparatus required to supply supercritical oxygen.

Now by the use of this invention one can supplement the oxygen provided to industrial scale oxidation reaction wherein ambient air is the major oxygen source, with greater operating efficiency, reduced operating costs and reduced capital costs than conventional supplemental oxygen systems.

Although the method of this invention has been described in detail with reference to certain embodiments, those skilled in the art will appreciate that other embodiments of the invention are contemplated within the spirit and scope of the claims.

**Claims**

1. A method for carrying out an oxidation reaction comprising:

(a) providing an air stream to a compressor;

(b) providing liquid oxygen into the air stream upstream of the compressor;

(c) vaporizing the liquid oxygen upstream of the compressor by heat exchange with the air to provide additional oxygen to the air and to cool and contract the air;

(d) compressing the cooled, contracted, oxygen enriched air by passage through the compressor;

(e) passing the compressed oxygen-enriched air in contact with a fluid having at least one oxidizable component; and

(f) reacting vaporized oxygen and oxygen from the air with oxidizable component(s) to carry out an oxidation reaction.

2. The method of Claim 1 wherein the heat exchange of step (c) is direct heat exchange.

3. The method of Claim 1 wherein the heat exchange of step (c) is indirect heat exchange.

4. The method of Claim 1 wherein the air is cooled by the vaporizing oxygen to a temperature which is still above the dew point of the air.

5. The method of Claim 1 wherein the air is cooled by the vaporizing oxygen to a temperature which is at or below the dew point of the air, and the resulting condensed water is removed from the air stream prior to passage into the compressor.

6. The method of Claim 1 wherein the oxidation reaction is carried out at a pressure exceeding 275 psia.

EP 0 368 288 A2